# EUROPEAN PATENT APPLICATION

(11) **EP 4 585 222 A1**
(43) Date of publication of application: **16.07.2025**
(21) Application number: 23862344.1
(22) Date of filing: 04.09.2023
(51) Int. Cl.: A61K 36/605, A61P 13/12, A61P 3/10, A61K 31/445, A61K 135/00, A61K 127/00, A61K 125/00

(54) **USE OF MULBERRY EXTRACT IN PREPARATION OF DRUG FOR PREVENTING AND/OR TREATING CHRONIC KIDNEY DISEASE**

(30) Priority: 08.09.2022 CN 202211093001; 16.01.2023 CN 202310057184
(71) Applicant: Beijing Wehand-Bio Pharmaceutical Co., Ltd, Beijing 102600 (CN); Guangxi Wehand-Bio Pharmaceutical Co., Ltd, Hechi, Guangxi 546300 (CN)
(72) Inventor: LIU, Yuling, Beijing 102600 (CN); JIN, Yiqun, Beijing 102600 (CN); ZHU, Xiangyang, Beijing 102600 (CN); LIU, Zhihua, Beijing 102600 (CN); HUANG, Mei, Beijing 102600 (CN); SHEN, Zhufang, Beijing 102600 (CN); LIU, Shuainan, Beijing 102600 (CN); LI, Caina, Beijing 102600 (CN); SONG, Jiawei, Beijing 102600 (CN); LIU, Quan, Beijing 102600 (CN); CAO, Hui, Beijing 102600 (CN); YE, Jun, Beijing 102600 (CN)
(74) Representative: ABG Intellectual Property Law, S.L.
(86) International application number: PCT/CN2023/116761
(87) International publication number: WO 2024/051650

(57) **Abstract**

Use of a mulberry extract in the preparation of a product for preventing and/or treating chronic kidney disease. The mulberry extract can improve renal function indexes of ZDF rats, improve serum oxidative/nitrative stress and inflammation-related indexes of the ZDF rats, reduce serum inflammatory factor levels in the ZDF rats, and improve renal pathological changes and renal fibrosis in the ZDF rats. The mulberry extract can also significantly reduce serum creatinine and urea nitrogen levels in CKD rats, and meanwhile, relieve the increase of kidney-to-body ratio.

## Description

### Technical Field

The invention is in the field of medicine, and particularly relates to use of a mulberry (*Morus alba* L.) extract in the preparation of a medicament for preventing and /or treating a chronic kidney disease.

### Background Art

The definition of a chronic kidney disease is abnormal kidney structure or function for ≥ 3 months (Chinese Journal of Nephrology, May 2022, Volume 38, Issue 5).

A chronic kidney disease (CKD) is a global public health problem, with an incidence rate up to 16%. Epidemiological statistics data show that the main causes of the chronic kidney disease are diabetes and hypertension. The Chronic kidney damage may further develop into kidney failure, inevitably requiring dialysis or kidney transplantation, thereby significantly reducing quality of life. However, the current treatment strategies for CKD mainly are focused on the aspects of controlling risk factors and alleviating symptoms, for example, angiotensin-converting enzyme inhibitors (ACEIs) and angiotensin II receptor antagonists (ARBs) can delay the progression of chronic kidney disease to a certain extent, but still cannot prevent its development, and may be accompanied by serious side effects, including coughing (using ACEIs) and severe hyperkalemia, which usually limits their use. Therefore, any new treatment methods that can prevent and/or treat a chronic kidney disease are of great significance.

### Summary of the Invention

The objective of the invention is to provide a new use of a mulberry extract or main active ingredients thereof as a medicament.

The new use of a mulberry extract or main active ingredients thereof as provided by the invention is any of (a1)-(a4) as follows:
(a1) use of the mulberry extract in the preparation of a medicament for treating a chronic kidney disease;
(a2) use of the mulberry extract in the preparation of a medicament for preventing a chronic kidney disease;
(a3) use of the mulberry extract in the treatment of a chronic kidney disease; and
(a4) use of the mulberry extract in the prevention of a chronic kidney disease.

The chronic kidney disease includes a diabetic kidney disease or a non-diabetic chronic kidney disease (such as a hypertensive kidney disease, a glomerulonephritis, a latent nephritis, a pyelonephritis, a Henoch-Schonlein purpura nephritis, a lupus nephritis, a gouty kidney, an IgA kidney disease, a nephrotic syndrome, a membranous kidney disease, a nephrotic syndrome, a polycystic kidney, etc.), that is, the chronic kidney disease is accompanied or not accompanied by diabetes.

The prevention and/or treatment of the chronic kidney disease is/are reflected in at least one of:
1) improvement in renal function indexes in a subject with the chronic kidney disease;
2) improvement in oxidative/nitrative stress and inflammatory status in a subject with the chronic kidney disease;
3) reduction in levels of renal inflammatory factors in a subject with the chronic kidney disease;
4) improvement in renal pathological changes in a subject with the chronic kidney disease;
5) improvement in renal fibrosis in a subject with the chronic kidney disease;
6) reduction in a trend of blood creatinine in a subject with the chronic kidney disease;
7) alleviation in a trend of increasing ratio of kidney weight to body weight in a subject with the chronic kidney disease; and
8) improvement in renal inflammation in a subject with the chronic kidney disease.

Further, the chronic kidney disease is a diabetic kidney disease;
The improvement in renal function indexes in the subject with the chronic kidney disease is reflected in at least one of: reduction in the level of blood urea nitrogen in the subject with the diabetic kidney disease; reduction in the contents of urinary albumin and β2-microglobulin in the subject with the diabetic kidney disease.

The improvement in oxidative/nitrative stress in the subject with the diabetic kidney disease is reflected in at least one of: a trend of increasing serum total antioxidant capacity (T-AOC) and superoxide dismutase (SOD) levels, significant reduction in serum inducible nitric oxide synthase (iNOS) and nitric oxide (NO) levels, reduction in renal mitochondrial lesion, and alleviation of mitochondrial damage in the subject with the diabetic kidney disease.

The improvement in inflammatory status in the subject with the diabetic kidney disease is specifically reflected in: the significant reduction in serum IL-1β and MCP-1 levels in the subject with the diabetic kidney disease.

Renal inflammatory factors include TNFα, IL-1β, IL-6, and C-reactive protein (CRP).

Renal pathological changes include at least one of: glomerular nodular sclerosis (with at least one confirmed KW nodule), varying degrees of renal interstitial fibrosis and inflammation, vascular hyalinization and sclerosis, and atrophy of renal tubular epithelial cells.

The renal fibrosis includes glomerular fibrosis and renal cystic fibrosis.

Further, the chronic kidney disease is a non-diabetic chronic kidney disease.

The improvement in renal function indexes in the subject with the non-diabetic chronic kidney disease is specifically reflected in: reduction in the levels of serum creatinine, serum urea nitrogen and serum uric acid in the subject with the chronic kidney disease.

The improvement in oxidative stress in the subject with the non-diabetic chronic kidney disease is specifically reflected in: improvement in the expression of SOD, CAT, and GSS, reduction of the level of MDA, reduction of renal mitochondrial lesion, and alleviation of mitochondrial damage in the subject with the chronic kidney disease.

The improvement in renal fibrosis in the subject with the non-diabetic chronic kidney disease is specifically reflected in: improvement in the expression of fibrosis related proteins in the subject with the chronic kidney disease; the fibrosis related proteins include at least one of: COL1A1, α-SMA, Fibronectine, and COL4A1.

The improvement in renal inflammation in the subject with the non-diabetic chronic kidney disease is specifically reflected in: inhibition of the infiltration of renal white blood cells and macrophages.

Renal pathological changes include at least one of the following aspects: atrophy or dilation of renal tubules, loss of brush borders, uneven thickening of renal basement membrane, and fusion or disappearance of podocytes.

The invention further protects use of a mulberry extract or main active ingredients thereof, which is at least one of (b1)-(b12) as follows:
(b1) preparation of a product for improving renal function indexes in a subject with a chronic kidney disease;
(b2) preparation of a product for improving oxidative/nitrative stress and inflammatory status in a subject with a chronic kidney disease;
(b3) preparation of a product for reducing levels of renal inflammatory factors in a subject with a chronic kidney disease;
(b4) preparation of a product for improving renal pathological changes in a subject with a chronic kidney disease;
(b5) preparation of a product for improving renal fibrosis in a subject with a chronic kidney disease;
(b6) preparation of a product for reducing a trend of blood creatinine in a subject with a chronic kidney disease;
(b7) improvement in renal function indexes in a subject with a chronic kidney disease;
(b8) improvement in oxidative/nitrative stress and inflammatory status in a subject with a chronic kidney disease;
(b9) reduction in levels of renal inflammatory factors in a subject with a chronic kidney disease;
(b10) improvement in renal pathological changes in a subject with a chronic kidney disease;
(b11) improvement in renal fibrosis in a subject with a chronic kidney disease; and
(b12) reduction in a trend of blood creatinine in a subject with a chronic kidney disease.

The product is a medicament or a pharmaceutical formulation.

The chronic kidney disease includes a diabetic kidney disease or a non-diabetic chronic kidney disease.

Further, the diabetes is type 2 diabetes.

The mulberry extract is a Ramulus Mori extract, a Cortex Mori extract and/or a Folium Mori extract. Alternatively, the mulberry extract can also be provided in the form of commercially available *Ramulus Mori* total alkaloid Tablet (NMPN Z20200002).

The mulberry extract can be prepared with reference to the method described in CN113143997A, and the detailed method for preparation comprises the steps of:
1) preparing a crude extract from a plant of *Moraceae*; and
2) separating the crude extract via a cation resin and/or optionally, an anion resin, to provide a mulberry extract.

The method can further comprise the steps of:
3) subjecting the effluent from the resin in step 2) to alcohol precipitation and collecting a supernatant; and
4) concentrating and drying the supernatant.

The method can further comprise the step of: concentrating and drying the effluent from the resin in step 2).

The mulberry extract acts on humans or mammals.

The plant of *Moraceae* can be selected from *Morus atropurpurea Roxb., Morus multicaulis Perr, Morus alba* (also known as White Mulberry), *Morus serrata Roxb., Morus mongolica Schneid* or Hybrid Mulberry, and the Hybrid Mulberry is preferably Yuesang No. 11, Guisangyou No. 62 or Sangteyou No. 2. Leaves, roots, twigs, barks, buds, stems, and fruits, among other parts, of the plant can be used.

In one embodiment of the invention, the mulberry extract mainly comprises alkaloids, and further polysaccharides, flavonoids, and amino acids.

Preferably, the alkaloids comprise at least one of 1-deoxynojirimycin or DNJ, N-methly-1-deoxynojirimycin, fagomine or FAG, 3-epi-fagomine, 1,4-dideoxy-1,4-imino-D-arabinitol or DAB, calystegin B2, calystegin C1, 2-O-(α -D-galactopyranosyl)-1-deoxynojirimycin, 6-O-(β-D-glucopyranosyl)-1-deoxynojirimycin, and 1,4-dideoxy-1,4-imino-(2-O-β -D-glucopyranosyl)-D-arabinitol.

Of these, the weight percentage of DNJ is not less than 50% of total alkaloids.

Based on the mulberry extract, each component has content by weight of:

| | |
|---|---|
| Alkaloids | 3-99%, |
| Polysaccharides | 0.2-70%, |
| Flavonoids | 0-10%, |
| Amino acids | 0-50%, |
| Other components | 0-25%; |

Preferably, based on the mulberry extract, each component has content by weight of:

| | |
|---|---|
| Alkaloids | 30-99%, |
| Polysaccharides | 0.2-35%, |
| Flavonoids | 0-2%, |
| Amino acids | 0-30%, |
| Other components | 0-20%; |

Preferably, based on the mulberry extract, each component has content by weight of:

| | |
|---|---|
| Alkaloids | 50-99%, |
| Polysaccharides | 0.2-35%, |
| Flavonoids | 0-2%, |
| Amino acids | 0-30%, |
| Other components | 0-20%; |

More preferably, based on the mulberry extract, each component has content by weight of:

| | |
|---|---|
| Alkaloids | 50-99%, |
| Polysaccharides | 0.2-25%, |
| Flavonoids | 0-1%, |
| Amino acids | 0-20%, |
| Other components | 0-20%. |

More preferably, based on the mulberry extract, each component has content by weight of:

| | |
|---|---|
| Alkaloids | 50-65%, |
| Polysaccharides | 20-25%, |
| Flavonoids | 0.5-1.5%, |
| Amino acids | 3-20%, |
| Other components | 8-20%. |

More preferably, based on the mulberry extract, each component has content by weight of:

| | |
|---|---|
| Alkaloids | 50-65%, |
| Polysaccharides | 20-25%, |
| Flavonoids | 0.5-1.5%, |
| Amino acids | 5-20%, |
| Other components | 8-20%. |

In one embodiment, the preparation of the mulberry extract comprises the steps of: preparing a crude extract; optionally, separating via a cation resin and/or an anion resin; optionally, subjecting the effluent from the resin to alcohol precipitation; and optionally, concentrating and drying. Preferably, the preparation of the mulberry extract comprises the steps of: step 1) preparing a crude extract; step 2) separating via a cation resin and/or optionally, an anion resin; optionally, step 3) subjecting the effluent from the resin in step 2) to alcohol precipitation; and optionally, step 4) concentrating and drying.

In one embodiment, the mulberry extract is prepared according to the steps of: crushing mulberry twigs (Ramulus Mori), mulberry leaves (Folium Mori) or mulberry root-barks (Cortex Mori), for extraction with water and/or alcoholic solution or acid water under heat reflux, with an amount of the solvent(s) 3-20 times the raw medicinal materials, repeating the extraction for 1-3 times, combing the extraction liquids, concentrating, going through a cation exchange resin, washing away with water (preferably, distilled water) the impurities that are not adsorbed, eluting with 0.2-3N aqueous ammonia, concentrating and subjecting the eluent to an anion exchange resin, collecting the portions that are not adsorbed, adding ethanol, removing impurities by precipitation, centrifuging, and subjecting the supernatant to concentration (preferably, concentration under reduced pressure) or drying (preferably, spray-drying or freeze-drying) to provide the extract.

In one embodiment, the mulberry extract is prepared according to the steps of: crushing mulberry twigs, mulberry leaves or mulberry root-barks for extraction with water and/or alcoholic solution or acid water under heat reflux, with an amount of the solvent(s) 3-20 times the raw medicinal materials, repeating the extraction for 1-3 times, combing the extraction liquids, concentrating, going through a cation exchange resin, washing away with water (preferably, distilled water) the impurities that are not adsorbed, eluting with 0.2-3N aqueous ammonia, concentrating and subjecting the eluent to an anion exchange resin, collecting the portions that are not adsorbed, and concentrating (preferably, concentrating under reduced pressure) or drying (preferably, spray-drying or freeze-drying) to provide the extract.

In one embodiment, the mulberry extract is prepared according to the steps of: crushing mulberry twigs, mulberry leaves or mulberry root-barks for extraction with water and/or alcoholic solution or acid water under heat reflux, with an amount of the solvent(s) 3-20 times the raw medicinal materials, repeating the extraction for 1-3 times, combing the extraction liquids, concentrating, going through a cation exchange resin, washing away with water (preferably, distilled water) the impurities that are not adsorbed, eluting with 0.2-3N aqueous ammonia, and subjecting the eluent to concentrating (preferably, concentrating under reduced pressure) or drying (preferably, spray-drying or freeze-drying) to provide the extract.

In one embodiment, the mulberry extract is prepared according to the steps of: crushing mulberry twigs, mulberry leaves or mulberry root-barks for extraction with water under heat reflux, with an amount of the solvent(s) 3-20 times (preferably 4-15 times, and more preferably 4-12 times) the raw medicinal materials, repeating the extraction for 1-3 times (extraction for preferably 0.5-3h each time, and more preferably 1-2h each time), combing the extraction liquids, concentrating, going through a cation exchange resin, washing away with water (preferably, distilled water) the impurities that are not adsorbed, eluting with 0.2-3N aqueous ammonia, subjecting the eluent to an anion exchange resin, collecting the portions that are not adsorbed (i.e., the effluent from the anion resin), adding ethanol, removing impurities by precipitation, centrifuging, and subjecting the supernatant to concentration (preferably, concentration under reduced pressure) or drying (preferably, spray-drying or freeze-drying) to provide the extract.

Preferably, the column is subjected to activation by washing with acidic solution, washing with alkaline solution, and washing with acidic solution in sequence after it is packed with a cation resin. Preferably, washing with alkaline solution is continued until the pH of the effluent is 8.0-9.5, preferably 8.5-9.5; preferably, the alkaline solution is selected from aqueous ammonia solution, sodium hydroxide solution, potassium hydroxide solution or sodium carbonate solution; preferably, the concentration of the alkaline solution is 0.5-4mol/L. Preferably, washing with acidic solution is continued until the pH of the effluent is 3.0-7.0, preferably 4.5-6.5. Preferably, the acidic solution is selected from hydrochloric acid solution (the concentration can be 1.5-2mol/L), phosphoric acid solution, and sodium phosphate dibasic-citric acid buffer. Optionally, the cation resin can further be subject to rinsing with 3-5 column volumes of deionized water following the last washing with acidic solution.

Preferably, the cation resin is 732-type strongly acidic styrene based cation exchange resin, 002SC-type strongly acidic styrene based cation resin, 734-type strongly acidic styrene based cation exchange resin, D001-type macroporous strongly acidic styrene based cation exchange resin or D113-type macroporous weakly acidic cation resin.

Preferably, the ratio of the amount of the cation resin used to the weight of the feed of the plant as raw materials is 1:2-20 (more preferably, the ratio of the amount of the cation resin used to the weight of the feed of the plant as raw materials is 1:2-7). After the crude extract from the plant is loaded onto a cation resin, the loaded cation resin is eluted with an eluent, preferably at a concentration of 0.5-2.5mol/L. Preferably, the eluent has a flow rate of 5-10BV/h, and more preferably, 5-6BV/h. The eluent is not collected until the effluent from the cation column is detected to have pH>7 and the collected liquid is subject to direct purification by an anion column.

Preferably, the column is subject to activation by washing with alkaline solution, washing with acidic solution, and washing with alkaline solution in sequence after it is packed with an anion resin. Preferably, washing with alkaline solution is continued until the pH of the effluent is 8.0-9.5, preferably 8.5-9.5; preferably, the alkaline solution is selected from the group consisting of aqueous ammonia solution, sodium hydroxide solution, potassium hydroxide solution or sodium carbonate solution; preferably, the concentration of the alkaline solution is 0.5-4mol/L. Preferably, washing with acidic solution is continued until the pH of the effluent is 3.0-7.0, preferably 3.5-4.5. Preferably, the acidic solution is selected from hydrochloric acid solution (the concentration can be 1.5-2mol/L), phosphoric acid solution, and sodium phosphate dibasic-citric acid buffer.

Preferably, the anion resin is 711-type strongly alkaline styrene based anion resin, 717- type strongly alkaline styrene based anion exchange resin, D201-type macroporous strongly alkaline styrene based anion exchange resin or D218-type macroporous strongly alkaline acrylic based anion exchange resin. Preferably, the ratio of the amount of the anion resin used to the weight of the feed of the plant as raw materials is 1:1-32 (more preferably, the ratio of the amount of the anion resin used to the weight of the feed of the plant as raw materials is 1:5-16). Collection begins when liquid flows out of the anion resin (preferably, the effluent with pH greater than 8 is collected). Preferably, collection is stopped when the volume of the collected liquid is 0.1-5 times the weight of the feed of the plant as raw materials.

Preferably, the ratio of ethanol used for treatment with alcohol precipitation to the weight of the feed of the plant as raw materials is 1:20-300 (more preferably, 1: 20-50). In the treatment with alcohol precipitation, stirring is performed at a speed of 40-500 rpm. The treatment with alcohol precipitation is for 12-24h.

Further, steps of impurity removal from the effluent from the anion resin by centrifugation or by filtration through microfiltration membranes, followed by concentration through reverse osmosis membranes are comprised prior to the treatment with alcohol precipitation. The specific gravity of the concentrated liquid can be 1.1-1.25.

Preferably, the medicament further comprises a pharmaceutically acceptable carrier. The carrier is a non-active ingredient with no toxic or harmful effects on human bodies and suitable for administration routes or administration manners. The carrier can be a solid or liquid auxiliary material. A solid auxiliary material includes, for example, microcrystalline cellulose, mannitol, lactose, pregelatinized starch, low-substituted hydroxypropyl cellulose, cross-linked povidone, sodium carboxymethyl starch, aspartame, calcium hydrogen phosphate, sodium lactate, poloxamer, sodium dodecyl sulfate, sodium carboxymethyl cellulose, gelatin, xanthan gum, povidone, starch, magnesium stearate, sodium carboxymethyl starch and powdered talc; and a liquid auxiliary material includes, for example, water, ethanol, syrup and glycerol.

Preferably, the medicament is in a dosage form of oral administration; further preferably, the medicament is a tablet, a capsule, an oral solution, an oral emulsion, a pill, a granule, a syrup, and a powder.

Compared with the existing technology, the present invention has the following advantages:
The mulberry extract with specific composition provided by the invention can effectively treat chronic kidney diseases, including a diabetic kidney disease.

### Brief Description of the Drawings

In order to explain the specific embodiments in the invention or the technical solutions in the prior art more clearly, the accompanying figures required for the description of the specific embodiments or the prior art will now be briefly introduced below. It is evident that the accompanying figures in the following description are some of the embodiments in the invention. It is possible for one those skilled in the art to get access to other figures according to these accompanying figures without doing creative work.

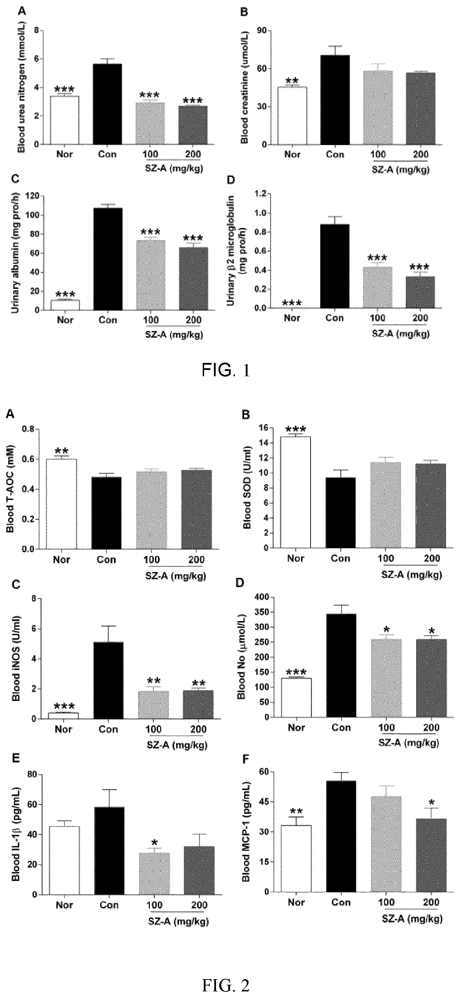

Fig. 1 shows the effect of long-term administration of SZ-A on renal function related indexes in ZDF rats in Experimental Example 1, where A represents serum urea nitrogen, B represents serum creatinine, C represents urinary albumin, and D represents urinary β 2-microglobulin, with ***p<0.001,**p<0.01 vs Con, mean ± SE, and n=8-10.
Fig. 2 shows the effect of long-term administration of SZ-A on oxidative/nitrative stress and inflammation in the bodies of ZDF rats in Experimental Example 1, where A represents serum total antioxidant capacity, B represents serum superoxide dismutase, C represents serum inducible nitric oxide synthase, D represents serum nitric oxide, E represents serum interleukin-1 β , and F represents serum monocyte chemotactic factor, with ***p<0.001, **p<0.01, *p<0.05 vs Con, mean ± SE, and n=8-10.

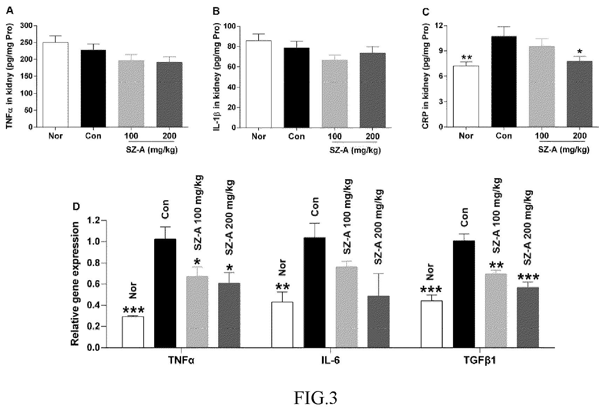

Fig. 3 shows the effect of long-term administration of SZ-A on renal inflammation in ZDF rats in Experimental Example 1, where A represents the level of renal tumor necrosis factor α, B represents the level of renal interleukin-1β, C represents the level of renal C-reactive protein, and D represents the gene expression of renal tumor necrosis factor α, interleukin-6, and transforming growth factor β1, with ***p<0.001, **p<0.01, *p<0.05 vs Con, mean ± SE, n=8-10 for A-C and 3-5 for D.

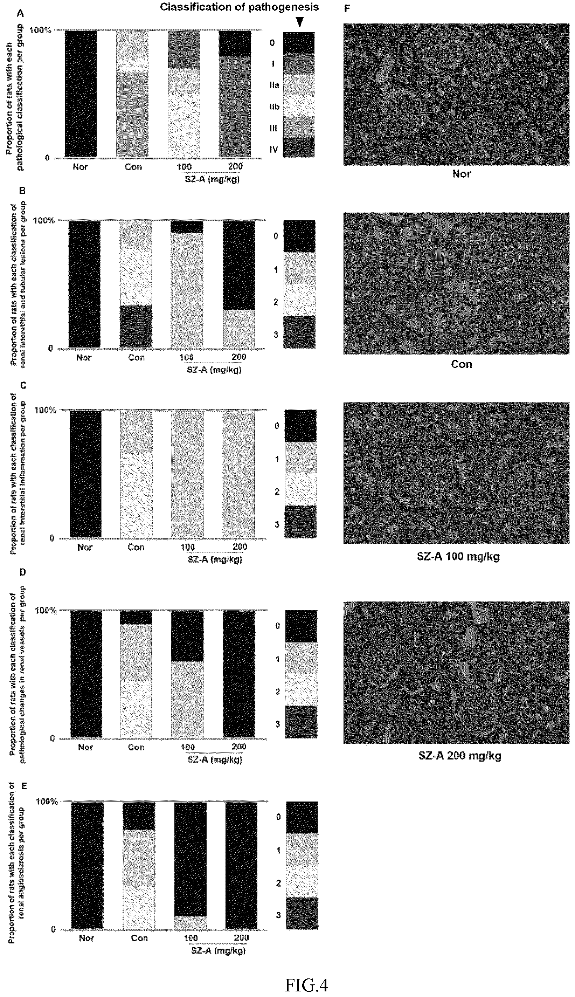

Fig. 4 shows the effect of long-term administration of SZ-A on renal pathological changes in ZDF rats in Experimental Example 1, where A represents pathological classification: 0, normal; I, mild or non-specific thickening of the glomerular basement membrane; IIa, mild glomerular mesangial hyperplasia; IIb, severe glomerular mesangial hyperplasia; III, glomerular nodular sclerosis (with at least one confirmed KW nodule); IV, advanced diabetic glomerulosclerosis (glomerulosclerosis >50%); B represents renal interstitial and tubular lesions: the higher the score in the legend, the more severe; C represents renal interstitial inflammation: the same as B; D represents vascular hyalinization: the same as B; E represents vascular sclerosis: the same as B; and F represents HE staining images, 200x.

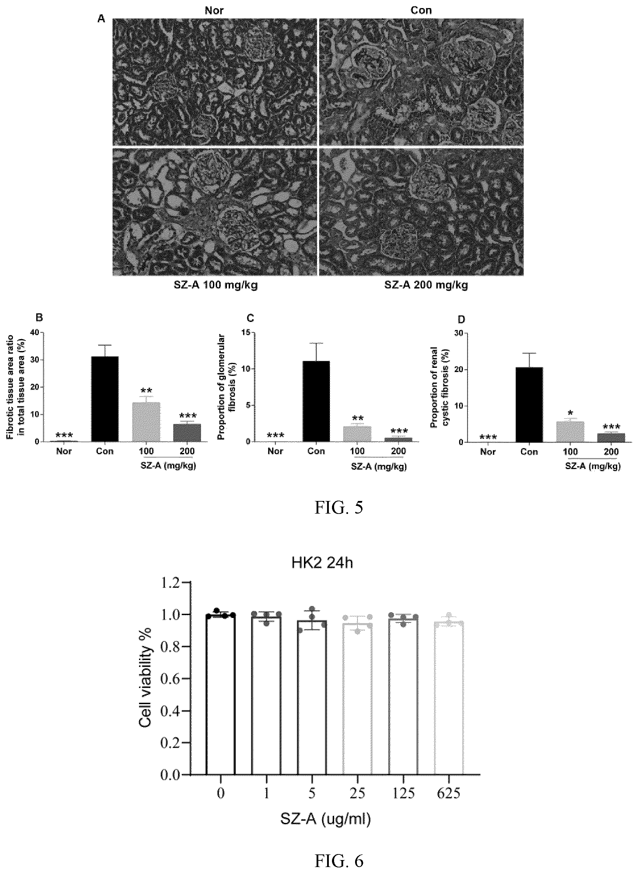

Fig. 5 shows the effect of long-term administration of SZ-A on renal fibrosis in ZDF rats in Experimental Example 1, where A represents Masson staining images, 200x; B represents the relative percentage of renal fibrosis area; C represents the proportion of glomerular fibrosis; and D represents the proportion of renal cystic fibrosis, with ***p<0.001, **p<0.01, *p<0.05 vs Con, mean ± SE, and n=8-10.
Fig. 6 shows the effect of cellular level SZ-A on the activity of human renal tubular epithelial cells in Experimental Example 3; mean ± SD, and n=4.

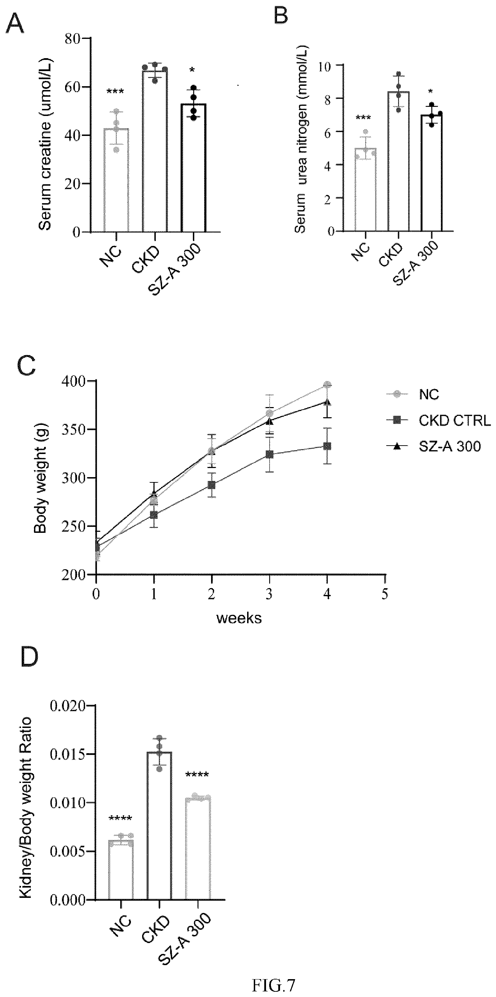

Fig.7 shows the effect of long-term administration of SZ-A on renal function related indexes in adenine model rats with a chronic kidney disease in Experimental Example 3, where A represents serum creatinine, B represents serum urea nitrogen, C represents rat body weight, and D represents rat kidney to body ratio, with ****p<0.0001, ***p<0.001, **p<0.01 vs Con, mean± SD, and n=4.

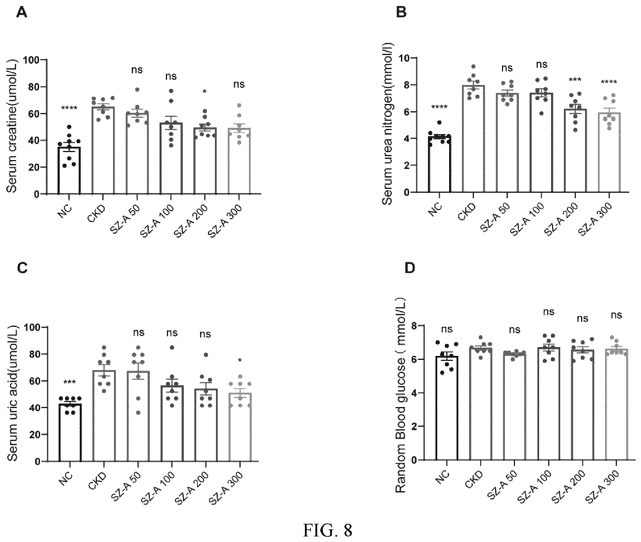

Fig. 8 shows the effect of long-term administration of SZ-A on serum creatinine, urea nitrogen, and uric acid levels in CKD rats in Experimental Example 4, with (A) serum creatinine, (B) serum urea nitrogen, (C) serum uric acid, (D) random blood glucose, mean ± SEM, n=8, ***p<0.001, **p<0.01, *p<0.05 vs CKD, and ns indicates no significant difference.

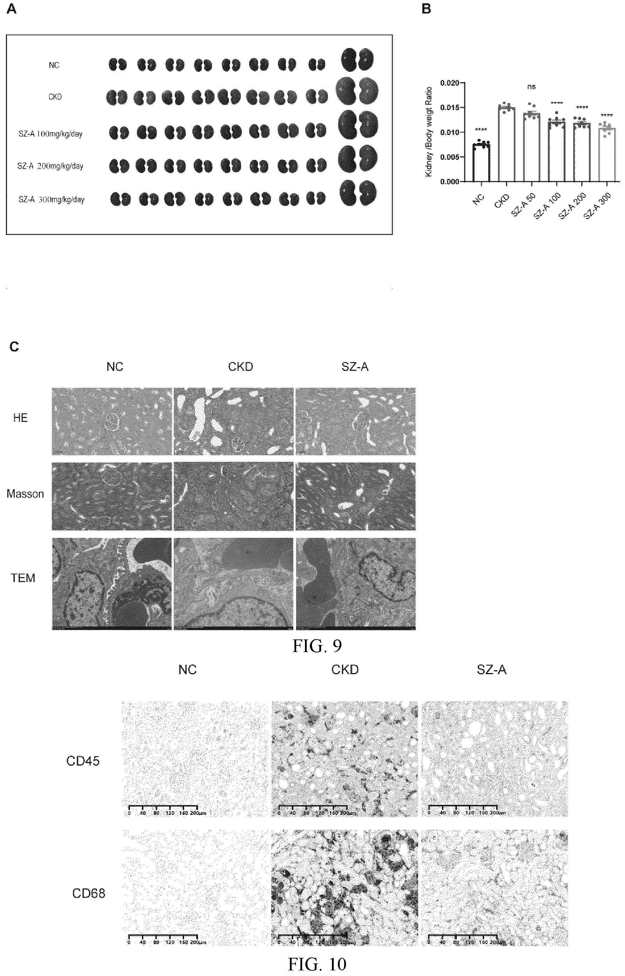

Fig. 9 shows the effect of long-term administration of SZ-A on kidney structure and kidney to body ratio in CKD rats in Experimental Example 4, with (A) kidney gross image, scale: 2cm, (B) kidney to body ratio, mean ± SEM, n=8-10, ***p<0.001, **p<0.01, *p<0.05 vs CKD, (C) HE, Masson staining, scale: 0.05mm; transmission electron microscopy 0.2 µ m.
Fig.10 shows the improvement of leukocyte and macrophage infiltration in the kidneys of CKD rats by long-term administration of SZ-A in Experimental Example 4, scale: 0.2mm.

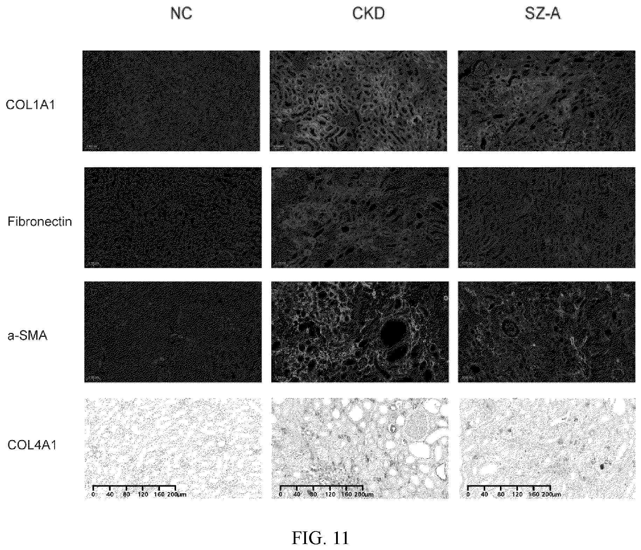

Fig. 11 shows the improvement of fibrosis related protein expression in CKD rats by long-term administration of SZ-A in Experimental Example 4, COL1A1, α-SMA, Fibronectine, scale: 0.01mm; COL4A1, scale: 0.2mm.

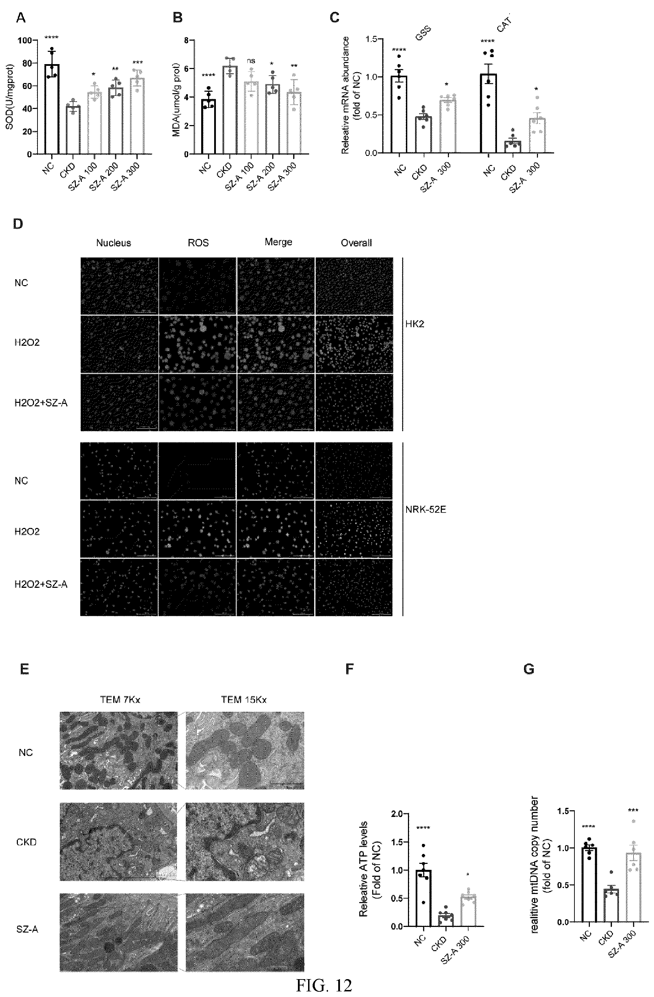

Fig. 12 shows the improvement of renal oxidative stress and mitochondrial function in CKD rats by long-term administration of SZ-A in Experimental Example 4, (A) SOD levels in renal tissue and (B) MDA levels in renal tissue, with mean ± SEM, n=5, ***p<0.001, **p<0.01, *p<0.05 vs CKD, (C) mRNAexpression levels of GSS and CAT in renal tissues of rats detected by RTqPCR, with mean ± SEM, n=6, ***p<0.001, **p<0.01, *p<0.05 vs CKD, (D) SZ-A improves ROS production in H₂O₂-induced HK2 and NRK-52E cells, (E) SZ-A improves the quantity, arrangement, and structure of mitochondria in kidneys of CKD rats, 7000x, scale: 2 µm, 15000x, scale: 1 µm, (F) SZ-A increases ATP content and (G) mitochondrial copy number in renal tissues of CKD rats, with mean ± SEM, n = 6,***p<0.001,**p<0.01, *p<0.05 vs CKD.

### Detailed Description of Examples

The invention will now be further explained in detail by way of examples. The features and advantages of the invention will be more explicit by virtue of these exemplary illustrations. The invention, however, is not limited to the following examples. Unless otherwise specified, the methods as described are all conventional ones. Unless otherwise specified, the raw materials as described would be commercially available.

As used herein, the professional language "exemplary" means "serving as an example, embodiment or illustration". Any of the examples described herein as "exemplary" is not necessarily to be construed as being superior or better than other examples.

In addition, the technical features involved in different embodiments of the invention described below can be combined with each other unless they conflict.

The contents of the components involved in the invention have been detected according to the published methods (with reference to the methods described in Patent Publications Nos.: CN111077247A and CN110393738A).

### I. Preparative Examples of Mulberry Extract

### Preparative Example 1

1000kg of fresh mulberry twigs (Yuesang No. 11, *Morus serrata Roxb.*) were taken for crushing, followed by addition of 4000L of water for extraction under heat reflux for 2h. The extraction liquids were combined and filtered to remove insoluble substances to provide a crude extract. The crude extract was thermally concentrated until the solid content reached 4% by mass, which was kept at 50°C as a loading liquid onto a cation resin column.

150kg of D113-type macroporous weakly acidic phenylpropene based cation resin was packed in a column, followed by washing with 2mol/L hydrochloric acid solution until the pH of the effluent was 4.5; washing with 1mol/L sodium hydroxide solution until the pH of the effluent was 8.5; washing with 2mol/L hydrochloric acid solution until the pH of the effluent was 4.5; and rinsing with 5 column volumes of deionized water to complete activation. The concentrated extraction liquids were loaded, followed by elution with 1000L of 2.5mol/L aqueous ammonia at an elution rate of 6BV/h. The eluent was collected when the effluent from the cation column was detected to have pH>7. Collection was stopped when the collected liquid reached 900L. The collected liquid was subject to direct purification by an anion column.

62.5kg of D218-type macroporous strongly alkaline acrylic based anion resin was packed in a column, followed by washing with 1.5mol/L sodium hydroxide solution until the pH of the effluent was 9.0; washing with 1.5mol/L hydrochloric acid solution until the pH of the effluent was 3.5; washing with 1.5mol/L sodium hydroxide solution until the pH of the effluent was 9.0, to complete activation. The collected eluent from the cation resin was loaded onto an anion resin. Collection of the effluent was not stopped until it reached 870L.

The collected liquid was centrifuged to remove impurities, and then concentrated through reverse osmosis membranes. As a result, the concentrated liquid had a specific gravity of 1.25 and was transferred to a tank for alcohol precipitation, to which 25L of anhydrous ethanol was added at 500rpm with a stirring paddle. Once the addition of ethanol was finished, stirring was stopped for alcohol precipitation for 24h. Supernatant was removed and concentrated under reduced pressure to provide an extractum as a mulberry twig extract (that is, SZ-A extract).

In the extractum as a mulberry twig extract, the content of alkaloids was 52%, polysaccharides 22%, flavonoids 0.8%, and amino acids 20%. In the alkaloids, the content of 1-DNJ was 60%, FAG 17%, and DAB 15%.

### Preparative Example 2

10kg of fresh mulberry twigs (Sangteyou No.2) were taken for crushing, followed by addition of 150L of water in two portions for extraction for 3h each time according to a decoction method. The extraction liquids were combined and filtered to remove insoluble substances. The extraction liquids were thermally concentrated until the solid content reached 8% by mass, and then transferred to a tank for alcohol precipitation, to which 2367.9g of anhydrous ethanol (3L) was added at 300rpm with a stirring paddle. Once the addition of ethanol was finished, stirring was stopped for alcohol precipitation for 24h. Supernatant was removed as a loading liquid onto a cation resin column. 5kg of 002SC-type strongly acidic styrene based cation resin was packed in a column, followed by activation of the cation resin according to the method of Preparative Example 1. The extraction liquids that were subject to concentration and alcohol precipitation were loaded, followed by elution with 100L of 5mol/L potassium chloride at an elution rate of 5BV/h. The effluent was detected with 20% silicotungstic acid. Collection began when white precipitate was generated. Collection was stopped when the collected liquid reached 25L, and the collected liquid was subject to direct purification by an anion column.

10kg of 711-type strongly alkaline styrene based anion resin was packed in a column, and the anion resin was subject to activation according to the method in Preparative Example 1. The collected eluent from the cation resin was loaded onto an anion resin. Collection of the effluent was not stopped until it reached 15L. The collected liquid was reloaded onto a cation resin and separated twice with a cation resin and an anion resin in sequence according to the above method.

The collected liquid obtained from column separation for three times was centrifuged to remove impurities, and then concentrated through reverse osmosis membranes. The concentrated liquid had a specific gravity of 1.25 and was transferred to a tank for alcohol precipitation, to which 125g of anhydrous ethanol was added at 1000rpm with a stirring paddle. Once the addition of ethanol was finished, stirring was stopped for alcohol precipitation for 24h. Supernatant was removed and concentrated under reduced pressure to provide an extractum as a mulberry twig extract. Fresh mulberry root-barks and mulberry leaves (Sangteyou No.2) were taken for additional extraction, the extraction method and parameters were same as above.

In the resulting extractum as a mulberry twig extract, the content of alkaloids was 98%, polysaccharides 0.2%, flavonoids 0.05%, and amino acids 0%. In the alkaloids, the content of 1-DNJ was 99%, FAG 0.5%, and DAB 0.4%.

In the resulting mulberry root-bark extract, the content of alkaloids was 95%, polysaccharides 2%, flavonoids 0.1%, and amino acids 1%. In the alkaloids, the content of 1-DNJ was 96%, FAG 1.5%, and DAB 1.4%.

In the resulting mulberry leaf extract, the content of alkaloids was 90%, polysaccharides 4%, flavonoids 0.1%, and amino acids 3%. In the alkaloids, the content of 1-DNJ was 91%, FAG 3.1%, and DAB 2.8%.

### Preparative Example 3

1000kg of fresh mulberry twigs (*Morus atropurpurea Roxb.* ) were taken for crushing, followed by addition of 11500L of water for extraction under heat reflux for 2h. The extraction liquids were combined and filtered to remove insoluble substances to provide a crude extract. The crude extract was first subject to centrifugation for impurity removal, and then to concentration through reverse osmosis membranes until the solid content reached 1% by mass as a loading liquid onto a cation resin column.

300kg of D001-type macroporous strongly acidic styrene based cation resin was packed in a column, and the cation resin was subjected to activation according to the method in Preparation Example 1. The concentrated crude extract was loaded, followed by elution with 5000L of 0.04mol/L ammonium nitrate at an elution rate of 5BV/h. The effluent was detected with 20% silicotungstic acid. Collection began when white precipitate was generated. Collection was stopped when the collected liquid reached 1000L.

The collected liquid obtained from cation column separation was concentrated through nanofiltration membranes, and concentrated under reduced pressure to provide an extractum of the extract.

In the resulting mulberry twig extract, the content of alkaloids was 15%, polysaccharides 20%, flavonoids 7%, and amino acids 45%. In the alkaloids, the content of 1-DNJ was 55%, FAG 23%, and DAB 10%.

### Preparative Example 4

333kg of dry mulberry twigs (Yuesang No.11) were taken for crushing, followed by addition of 4000L of water for extraction in two portions under heat reflux, with each reflux for 1h. The extraction liquids were combined, filtered, and concentrated to 1kg of crude drug/L.

150kg of D113-type macroporous weakly acidic phenylpropene based cation resin was packed in a column, followed by washing with 2mol/L hydrochloric acid solution until the pH of the effluent was 4.5; washing with 1mol/L sodium hydroxide solution until the pH of the effluent was 8.5; washing with 2mol/L hydrochloric acid solution until the pH of the effluent was 4.5; and rinsing with 5 column volumes of deionized water to complete activation. The concentrated extraction liquids were loaded, followed by elution with 1000L of 2.5mol/L aqueous ammonia at an elution rate of 6BV/h. The eluent was collected when the effluent from the cation column was detected to have pH>7. Collection was stopped when the collected liquid reached 900L. The collected liquid was subject to direct purification by an anion column.

125kg of D218-type macroporous strongly alkaline acrylic based anion resin was packed in a column, followed by washing with 1.5mol/L sodium hydroxide solution until the pH of the effluent was 9.0; washing with 1.5mol/L hydrochloric acid solution until the pH of the effluent was 3.5; washing with 1.5mol/L sodium hydroxide solution until the pH of the effluent was 9.0, to complete activation. The collected eluent from the cation resin was loaded onto an anion resin. Collection of the effluent having a pH>8 was not stopped until it reached 870L.

The collected liquid obtained from anion column separation was subject to filtration through microfiltration membranes to remove impurities, and then concentrated through reverse osmosis membranes. The concentrated liquid had a specific gravity of 1.1 and was transferred to a tank for alcohol precipitation, to which 15kg of anhydrous ethanol was added at 400rpm with a stirring paddle. Once the addition of ethanol was finished, stirring was stopped for alcohol precipitation for 24h. Supernatant was removed and concentrated under reduced pressure to provide an extractum as a mulberry twig extract. Contents in the sample are alkaloids 80%, polysaccharides 5%, flavonoids 0.1%, and amino acids 4%. In the alkaloids, the content of 1-DNJ was 75%, FAG 12%, and DAB 10%.

### Preparative Example 5

400kg of dry mulberry twigs (Yuesang No.11) were taken for crushing, followed by addition of 4000L of water for extraction in two portions under heat reflux, with each reflux for 1h. The extraction liquids were combined, filtered, and concentrated to 1kg of crude drug/L.

62.5kg of D218-type macroporous strongly alkaline acrylic based anion resin was packed in a column, followed by washing with 1.5mol/L sodium hydroxide solution until the pH of the effluent was 9.0; washing with 1.5mol/L hydrochloric acid solution until the pH of the effluent was 3.5; washing with 1.5mol/L sodium hydroxide solution until the pH of the effluent was 9.0, to complete activation. The collected, extracted concentrate was loaded onto an anion resin, and the effluent was collected.

The collected liquid obtained from anion column separation was subject to filtration through microfiltration membranes to remove impurities, and then concentrated through reverse osmosis membranes, and further concentrated under reduced pressure and dried to provide an extractum as a mulberry twig extract. Contents in the sample are alkaloids 3%, polysaccharides 70%, flavonoids 10%, and amino acids 10%. In the alkaloids, the content of 1-DNJ was 68%, FAG 17%, and DAB 8%.

### Preparative Example 6

1500kg of fresh mulberry twigs (Yuesang No. 11, *Morus serrata Roxb.*) were taken for crushing, followed by addition of 6000L of water for extraction under heat reflux for 2h. The extraction liquids were combined and filtered to remove insoluble substances to provide a crude extract. The crude extract was thermally concentrated until the solid content reached 4% by mass, which was kept at 50°C as a loading liquid onto a cation resin column.

100kg of D113-type macroporous weakly acidic phenylpropene based cation resin was packed in a column, followed by washing with 2mol/L hydrochloric acid solution until the pH of the effluent was 4.5; washing with 1mol/L sodium hydroxide solution until the pH of the effluent was 8.5; washing with 2mol/L hydrochloric acid solution until the pH of the effluent was 4.5; and rinsing with 5 column volumes of deionized water to complete activation. The concentrated extraction liquids were loaded, followed by elution with 1000L of 2.5mol/L aqueous ammonia at an elution rate of 6BV/h. The eluent was collected when the effluent from the cation column was detected to have pH>7. Collection was stopped when the collected liquid reached 900L. The collected liquid was subject to direct purification by an anion column.

62.5kg of D218-type macroporous strongly alkaline acrylic based anion resin was packed in a column, followed by washing with 1.5mol/L sodium hydroxide solution until the pH of the effluent was 9.0; washing with 1.5mol/L hydrochloric acid solution until the pH of the effluent was 3.5; washing with 1.5mol/L sodium hydroxide solution until the pH of the effluent was 9.0, to complete activation. The collected eluent from the cation resin was loaded onto an anion resin. Collection of the effluent was not stopped until it reached 870 L. The effluent was concentrated under reduced pressure to provide an extractum as a mulberry twig extract, in which the content of alkaloids was 30%, polysaccharides 35%, flavonoids 2%, and amino acids 25%. In the alkaloids, the content of 1-DNJ was 62%, FAG 20%, and DAB 13%.

### Preparative Example 7

1000kg of fresh mulberry twigs (Yuesang No. 11, *Morus serrata Roxb.*) were taken for crushing, followed by addition of 4000L of water for extraction under heat reflux for 2h. The extraction liquids were combined and filtered to remove insoluble substances to provide a crude extract. The crude extract was thermally concentrated until the solid content reached 4% by mass, which was kept at 50°C as a loading liquid onto a cation resin column.

100kg of D113-type macroporous weakly acidic phenylpropene based cation resin was packed in a column, followed by washing with 2mol/L hydrochloric acid solution until the pH of the effluent was 4.5; washing with 1mol/L sodium hydroxide solution until the pH of the effluent was 8.5; washing with 2mol/L hydrochloric acid solution until the pH of the effluent was 4.5; and rinsing with 5 column volumes of deionized water to complete activation. The concentrated extraction liquids were loaded, followed by elution with 1000L of 2.5mol/L aqueous ammonia at an elution rate of 6BV/h. The eluent was collected when the effluent from the cation column was detected to have pH>7. Collection was stopped when the collected liquid reached 900L. The collected liquid was subject to direct purification by an anion column.

62.5kg of D218-type macroporous strongly alkaline acrylic based anion resin was packed in a column, followed by washing with 1.5mol/L sodium hydroxide solution until the pH of the effluent was 9.0; washing with 1.5mol/L hydrochloric acid solution until the pH of the effluent was 3.5; washing with 1.5mol/L sodium hydroxide solution until the pH of the effluent was 9.0, to complete activation. The collected eluent from the cation resin was loaded onto an anion resin. Collection of the effluent was not stopped until it reached 870 L. The effluent was concentrated under reduced pressure to provide an extractum as a mulberry twig extract, in which the content of alkaloids was 40%, polysaccharides 25%, flavonoids 0.5%, and amino acids 25%. In the alkaloids, the content of 1-DNJ was 57%, FAG 24%, and DAB 16%.

### Preparative Example 8

333kg of dry mulberry twigs (Yuesang No.11) were taken for crushing, followed by addition of 4000L of water for extraction in two portions under heat reflux, with each reflux for 1h. The extraction liquids were combined, filtered, and concentrated to 1kg of crude drug/L.

150kg of D113-type macroporous weakly acidic phenylpropene based cation resin was packed in a column, followed by washing with 2mol/L hydrochloric acid solution until the pH of the effluent was 4.5; washing with 1mol/L sodium hydroxide solution until the pH of the effluent was 8.5; washing with 2mol/L hydrochloric acid solution until the pH of the effluent was 4.5; and rinsing with 5 column volumes of deionized water to complete activation. The concentrated extraction liquids were loaded, followed by elution with 1000L of 2.5mol/L aqueous ammonia at an elution rate of 6BV/h. The eluent was collected when the effluent from the cation column was detected to have pH>7. Collection was stopped when the collected liquid reached 900L. The collected liquid was subject to direct purification by an anion column.

62.5kg D218-type macroporous strongly alkaline acrylic based anion resin was packed in a column, followed by washing with 1.5mol/L sodium hydroxide solution until the pH of the effluent was 9.0; washing with 1.5mol/L hydrochloric acid solution until the pH of the effluent was 3.5; washing with 1.5mol/L sodium hydroxide solution until the pH of the effluent was 9.0, to complete activation. The collected eluent from the cation resin was loaded onto an anion resin. Collection of the effluent having a pH>8 was not stopped until it reached 870L.

The collected liquid obtained from anion column separation was subject to filtration through microfiltration membranes to remove impurities, and then concentrated through reverse osmosis membranes. The concentrated liquid had a specific gravity of 1.1 and was transferred to a tank for alcohol precipitation, to which 15kg of anhydrous ethanol was added at 400rpm with a stirring paddle. Once the addition of ethanol was finished, stirring was stopped for alcohol precipitation for 24h. Supernatant was removed and concentrated under reduced pressure to provide an extractum as a mulberry twig extract. Contents in the sample are alkaloids 63%, polysaccharides 23%, flavonoids 1%, and amino acids 5%. In the alkaloids, the content of 1-DNJ was 61.9%, FAG 16.6%, and DAB 11.1%.

### Experimental Example 1: Pharmacodynamic assays of mulberry extract in preventing and/or treating the diabetic kidney disease by long-term administration at the animal level

### I. Experimental Designs

### 1. Experimental Materials

### 1.1 Reagents

The creatinine detection kit (Nanjing Jiancheng Bioengineering Institute), urea nitrogen detection kit (Nanjing Jiancheng Bioengineering Institute), albumin detection kit (Abcam), β2-microglobulin detection kit (Beijing Lvyuan Bode Biotechnology Co., Ltd.), total antioxidant capacity (T-AOC), superoxide dismutase (SOD), inducible nitric oxide synthase (iNOS), and nitric oxide (NO) were all purchased from Nanjing Jiancheng Bioengineering Institute, tumor necrosis factor alpha (TNFα), interleukin-1β (IL-1β), monocyte chemotactic protein-1 (MCP-1), and C-reactive protein (CRP) were all purchased from Abcam, and TransStart^{®} The Tip Green qPCR SuperMix kit was purchased from Beijing TransGene Biotech.

### 1.2 Experimental Drugs

The mulberry twig extracts prepared in Preparative Example 1 were formulated at different concentrations and dissolved in water.

### 1.3 Experimental Animals

Spontaneous type 2 diabetes ZDF-Leprfa/Crl rats (fa/fa) and control rats (fa/+), male, 7 to 9 weeks old, purchased from Beijing Vital River Laboratory Animal Technology Co., Ltd., were raised in the SPF-grade animal house of Institute of Materia Medica, Chinese Academy of Medical Sciences, fed with special diet (Purina, 5008) (with free access to drinking water) for 2 months, to develop obvious diabetes, and the animal were continued to be fed for 3 months and then used in the experiment.

### 2. Experimental Methods

ZDF rats with spontaneous type 2 diabetes, divided into three groups, with 10 rats in each group, were administrated by oral gavage for nine consecutive weeks, and the specific experimental groupings, dosages and administration frequency are shown in Table 1. Meanwhile, the same litter of fa/+rats were used as controls, namely the normal control group (Nor, 8 rats). During the experiment, urine was collected using a metabolic cage, and the contents of urinary albumin and β2-microglobulin were measured using an ELISA kit. After the experiment, whole blood and kidney tissues were separated, and serum urea nitrogen and creatinine levels, as well as oxidative stress, nitrate stress, and inflammation related indexes were measured using reagent kits; an ELISA kit was used to measure inflammation related indexes in renal homogenate; real time quantitative PCR method was used to investigate the expression levels of inflammation and fibrosis related genes in the kidney. Meanwhile, kidney paraffin sections were prepared for HE staining and Masson staining.

**Table 1**

| Groups | Drugs | Dosages (by alkaloids) | Administration Frequency |
|---|---|---|---|
| Normal Control (Nor) | Water | - | Once a day |
| Model Group (Con) | Water | - | Once a day |
| SZ-A-low | SZ-A extract | 100 mg/kg/d | Once a day |
| SZ-A-high | SZ-A extract | 200 mg/kg/d | Once a day |

### II. Experimental Results

### 1. The effects of long-term administration of SZ-A on renal function related indexes

Compared with the Nor group, the levels of blood urea nitrogen and creatinine, as well as the levels of urinary albumin and β2-microglobulin in the kidneys of ZDF rats (Con) increased significantly, indicating that they developed obvious diabetic kidney disease. Compared with the Con group, long-term administration of SZ-A at doses of 100 and 200 mg/kg could significantly and dose dependently reduce blood urea nitrogen levels (P<0.001) in ZDF rats, while reducing serum creatinine levels by 17.3% and 19.5%, respectively (P>0.05); and long-term administration of SZ-A could also dose dependently reduce the contents of urinary albumin and urinary β2-microglobulin in ZDF rats (P<0.001) (Fig. 1).

### 2. The effects of long-term administration of SZ-A on overall oxidative/nitrative stress and inflammation in ZDF rats

Compared with the Nor group, the levels of serum antioxidant enzymes T-AOC and SOD in ZDF rats (Con) significantly decreased, while the levels of nitrate stress indexes, iNOS and NO, as well as inflammation related index, MCP-1, significantly increased, indicating that ZDF rats experienced oxidative/nitrative stress and inflammatory damage.

Compared with the Con group, long-term administration of SZ-A at doses of 100 and 200 mg/kg had a trend of increasing serum total antioxidant capacity (T-AOC) and superoxide dismutase (SOD) levels in ZDF rats, simultaneously significantly reducing serum inducible nitric oxide synthase (iNOS) and nitric oxide (NO) levels (P<0.05). In addition, long-term administration of SZ-A could significantly reduce the levels of serum IL-1β and MCP-1 in ZDF rats (P<0.05) (Fig. 2).

### 3. The effects of long-term administration of SZ-A on renal inflammation and fibrosis related indexes in ZDF rats

Compared with the Nor group, the levels of C-reactive protein (CRP) in the kidneys of ZDF rats (Con) significantly increased, indicating the occurrence of significant inflammatory infiltration. Compared with the Con group, long-term administration of SZ-A at doses of 100 and 200 mg/kg showed a trend of reducing TNFα and IL-1β in the kidneys of ZDF rats, and dose dependently lowering their renal CRP levels. The effect of 200 mg/kg was statistically significant (P<0.05). In addition, long-term administration of SZ-A at doses of 100 and 200 mg/kg significantly and dose dependently reduced the expression of TNFα and TGF β1 genes in the kidneys of ZDF rats (P<0.05), simultaneously having a trend of dose dependently reducing the expression of IL-6 gene in the kidneys of rats (P>0.05) (Fig. 3).

### 4. HE staining of kidneys in ZDF rat

Compared with the Nor group, ZDF rats (Con) showed significant glomerular nodular sclerosis (with at least one confirmed KW nodule), accompanied by varying degrees of renal interstitial fibrosis and inflammation, vascular hyalinization and sclerosis, as well as atrophy of renal tubular epithelial cells. Compared with the Con group, long-term administration of SZ-A at doses of 100 and 200 mg/kg could significantly improve the above pathological characteristics in ZDF rats, and there was a certain dose-effect relationship (Fig. 4).

The scoring criteria for B-D in Fig. 4 are shown in Table 2 below.

**Table 2**

| Lesions | Scores | Scoring Criteria |
|---|---|---|
| Renal interstitial and tubular lesions | 0 | No interstitial fibrosis or atrophy of renal tubular epithelial cells (IFTA) |
| | 1 | Interstitial fibrosis and atrophy of renal tubular epithelial cells (IFTA), <25% |
| | 2 | Interstitial fibrosis and atrophy of renal tubular epithelial cells (IFTA), 25%-50% |
| | 3 | Interstitial fibrosis and atrophy of renal tubular epithelial cells (IFTA), >50% |
| Renal interstitial inflammation | 0 | No |
| | 1 | Only inflammatory infiltration related to IFTA was observed |
| | 2 | There is also inflammatory infiltration in areas without IFTA |
| Vascular hyalinization | 0 | No vascular hyalinization |
| | 1 | Vascular hyalinization in one site |
| | 2 | Vascular hyalinization in more than one site |
| Vascular sclerosis | 0 | No intima thickening |
| | 1 | Intima thickened, not exceeding the thickness of the media |
| | 2 | Intima thickened, exceeding the thickness of the media |

### 5. Masson staining of kidneys in ZDF rat

Compared with the Nor group, the kidneys of ZDF rats (Con) showed significant fibrosis, and both glomeruli and renal capsules exhibited significant fibrosis characteristics. Compared with Con, long-term administration of SZ-A at doses of 100 and 200 mg/kg could dose dependently improve renal fibrosis in ZDF rats, with the effect of 200 mg/kg being statistically significant (P<0.001) (Fig. 5).

### Experimental Example 2: Pharmacodynamic assays of mulberry extract in preventing and/or treating the diabetic kidney disease at the cellular level

High-glucose cultured rat mesangial cells were treated with mulberry extracts from Preparative Examples 1 to 8. Further, the effects of mulberry extracts on mesangial cell proliferation, α-SAM expression, and synthesis of type IV collagen and fibronectin in mesangial cells were studied. The results showed that the mulberry extracts from above preparative examples at the same dosage had varying degrees of effects.

### Experimental Example 3: Pharmacodynamic assays of mulberry extract in preventing and/or treating the chronic kidney diseases (non-diabetic chronic kidney diseases) by long-term administration at animal level

### I. Experimental Designs

### 1. Experimental materials

**1.1 Reagents**
   Creatinine detection kit (Nanjing Jiancheng Bioengineering Institute), urea nitrogen detection kit (Nanjing Jiancheng Bioengineering Institute), CCK8 kit (Beiren Chemical Technology Co., Ltd.), adenine (sigma), DMEM/F-12 medium (Thermo Fisher Scientific), fetal bovine serum (Thermo Fisher Scientific).
**1.2 Experimental drugs**
   The mulberry twig extracts prepared in Preparative Example 1 were formulated at different concentrations and dissolved in water;
**1.3 Experimental animals**
   Wistar rats, male, 200g, purchased from Beijing Vital River Laboratory Animal Technology Co., Ltd., were raised in the SPF-grade animal house of Institute of Materia Medica, Chinese Academy of Medical Sciences. They were given free access to regular feed and water, and the experimental period was 4 weeks.
**1.4 Experimental cells**
   Human renal tubular epithelial cell HK2 (Cell Resource Center, Institute of Basic Medical Sciences, Chinese Academy of Medical Sciences)

### 2. Experimental methods

### Cell experiments:

HK2 cells in logarithmic growth phase were taken and inoculated into a 96 well-plate at a density of 1 × 10⁵ cells per milliliter, and incubated overnight at 37 °C. SZ-A (0-625ug/ml) prepared with PBS at different concentration gradients were added and incubated for 24 hours. Finally, the cells were washed twice with PBS and CCK-8 reagent was added. After incubation at 37 °C for 60 minutes, the absorbance at 450 nm was measured and the cell viability was calculated.

### Animal experiments:

Wistar male rats, divided into three groups, with four rats in each group, were administrated by oral gavage, the specific experimental groupings, dosages and administration frequency are shown in Table 3, and after one week of adaptive feeding, the rats were modelled and simultaneously administered for four consecutive weeks. After the experiment, whole blood and kidney tissues were separated, and serum urea nitrogen and creatinine levels were measured using kits.

**Table 3**

| Groups | Drugs | Dosages (by alkaloids) | Administration Frequency |
|---|---|---|---|
| Normal Control (Nor) | Water | - | Once a day |
| Model Group (Con) | Adenine | 100mg/kg/d | Once a day |
| SZ-A group | SZ-A extract | 300 mg/kg/d | Once a day |
| | Adenine | 100mg/kg/d | |

### II. Experimental results

### 1. The effects of SZ-A on the activity of in renal tubular epithelial cells HK2

The results are shown in Fig. 6. As shown in Fig. 6, compared with the control group, high concentrations of SZ-A could not inhibit the viability of HK2 cells.

### 2. The effect of long-term administration of SZ-A on renal function related indexes

The results are shown in Fig. 7. As shown in Fig. 7, compared with the Nor group, blood urea nitrogen and creatinine levels in CKD (Con) rats significantly increased (P<0.001), and kidney to body ratio significantly increased (P<0.0001), with a decrease in body weight, indicating the occurrence of significant chronic kidney disease. Compared with the Con group, long-term administration of SZ-A at a dose of 300 mg/kg could significantly reduce the levels of serum creatinine and urea nitrogen in CKD rats (P<0.05), and simultaneously alleviate the increase in kidney to body ratio (P<0.0001).

### Experimental Example 4: Pharmacodynamic assays of mulberry extract in preventing and/or treating the chronic kidney diseases (non-diabetic chronic kidney diseases) by long-term administration at animal level

### I. Experimental Designs

### 1. Experimental materials

### 1.1 Reagents

Creatinine detection kit, urea nitrogen detection kit, and uric acid detection kit (Nanjing Jiancheng Bioengineering Institute), CCK8 kit (Beiren Chemical Technology Co., Ltd.), adenine (sigma), DMEM/F-12 medium (Thermo Fisher Scientific), fetal bovine serum (Thermo Fisher Scientific), ATP detection kit (Beyotime), DNA extraction kit (QIAamp), ROS detection kit DCFH-DA (Solarbio), SOD detection kit, MDA detection kit (Beyotime), CD45, CD68, COL1A1, Fibronectine, α-SMA, COL4A1 primary antibody, secondary antibody fluorescent antibody (Abcam).

### 1.2 Experimental drugs

The mulberry twig extracts prepared in Preparative Example 1 were formulated at different concentrations and dissolved in water.

### 1.3 Experimental animals

Wistar rats, male, 200g, purchased from SPF (Beijing) Biotechnology Co., Ltd., were raised in the SPF-grade animal house of SINO ANIMAL (Beijing) Technology Co., Ltd. They were given free access to regular feed and water, and the experimental period was 5 weeks.

### 2. Experimental methods

### Animal experiments:

Wistar male rats, divided into 6 groups, with 8 rats in each group, were administrated by oral gavage, the specific experimental groupings, dosages and administration frequency are shown in Table 4, and after one week of adaptive feeding, the rats were modelled and simultaneously administered for four consecutive weeks. After the experiment, whole blood and kidney tissues were separated, and serum uric acid, urea nitrogen and creatinine levels were measured using kits.

**Table 4**

| Groups | Drugs | Dosages (calculated by alkaloids) | Administration Frequency |
|---|---|---|---|
| Normal Control(NC) | Purified water | - | Once a day |
| Model Group(Con) | Adenine | 100mg/kg/d | Once a day |
| SZ-A 50 group | Adenine+SZ-A extract | 100mg/kg/d+50 mg/kg/d | Once a day |
| SZ-A 100 group | Adenine+SZ-A extract | 100mg/kg/d+100 mg/kg/d | Once a day |
| SZ-A 200 group | Adenine+SZ-A extract | 100mg/kg/d+200 mg/kg/d | Once a day |
| SZ-A300 group | Adenine+SZ-A extract | 100mg/kg/d+300 mg/kg/d | Once a day |

### II. Experimental results

### 1. The effects of long-term administration of SZ-A on renal function related indexes

It can be seen from A-C in Fig. 8 that compared with the NC group, the levels of creatinine, urea nitrogen, and uric acid in the model group (CKD) of rats significantly increased, indicating the occurrence of significant chronic kidney disease. Compared with the model group, long-term administration of SZ-A at doses of 50, 100, 200, and 300 mg/kg can significantly reduce the levels of serum creatinine, urea nitrogen, and uric acid in ZDF rats, with a certain dose-dependent effect.

As shown in the random blood glucose measurement results of D in Fig. 8, there was no statistically significant difference in blood glucose levels among all groups, indicating that the improvement effect of SZ-A on the kidneys is independent of blood glucose.

### 2. The effects of long-term administration of SZ-A on renal physiological structure and fibrosis

As shown in A of Fig. 9, it was visible to the naked eye that the kidneys of the rats from the normal group were reddish brown in color with a smooth and rounded surface, while the model group is grayish-white with severe edema, increased volume, uneven surface, and diffuses white particles with the size of rice grains. Each SZ-A group could significantly improve the above symptoms in a dose-dependent manner, and reduce the kidney to body ratio (see B of Fig. 9).

As shown in C of Fig. 9, it could be seen under light microscopy that compared with the normal group, the renal tubules of the rats from the model group showed cystic dilation, and brown needle shaped crystals were deposited inside the renal tubules, forming crystalline tubules. A large number of monocytes and lymphocytes were infiltrated, accompanied by an aggravation of the degree of focal glomerular and tubular fibrosis. SZ-A can significantly improve the above pathological changes and alleviate renal tubular lesion and renal fibrosis in CKD rats.

As shown in C of Fig. 9, under transmission electron microscopy, the microstructure reveals uneven thickening of the renal basement membrane in CKD rats, widening of podocytes, and partial disappearance of fusion. SZ-A can improve the aforementioned lesions.

### 3. The effects of long-term administration of SZ-A on renal inflammation and fibrosis in CKD rats

As shown in Fig. 10, in terms of inflammation, the expression of renal leukocyte marker CD45 was significantly increased in the model group compared with the control rats with normal renal function, and treatment by administration of SZ-A 300mg/kd/day could significantly reduce the expression of CD45. Similarly, the expression of macrophage surface marker CD68 was significantly reduced in the kidneys of the SZ-A treatment group compared with the model group. This indicated that the infiltration of white blood cells and macrophages was inhibited, and inflammation was improved.

As shown in Fig. 11, the expression of renal fibrosis related proteins was also detected. The results showed that compared with the NC group, the model group had focal renal tubular and glomerular fibrosis, and the expression of renal fibrosis related proteins COL1A1, Fibronectine, a-SMA, and COL4A1 significantly increased. The treatment by administration of SZ-A 300mg/kg/day could significantly reduce the expression, proving that SZ-A could improve the progression of renal fibrosis in CKD rats.

### 4. The effects of long-term administration of SZ-A on renal oxidative stress and mitochondrial function in CKD rats

As shown in A-C of Fig. 12, enzymes and products related to oxidative stress in the kidney tissues of the rats were detected. The results showed that compared with the NC group, the activity of SOD with antioxidant activity in the kidneys of the model group rats was significantly reduced, and the gene expressions of H₂O₂ oxidase CAT and glutathione synthase GSS were significantly reduced; correspondingly, the peroxidation product MDA significantly increased, while SZ-A treatment could increase the expression of SOD, CAT, and GSS, reduce the level of MDA, and play a role in improving oxidative stress in the body.

The results of the cell experiment in D of Fig. 12 indicated that SZ-A can reduce the production of ROS (green fluorescence labelled) induced by H₂O₂ for both types of renal tubular epithelial cells, verifying the direct antioxidant stress effect.

In addition, the results of transmission electron microscopy (as shown in E of Fig. 12) showed that the number of mitochondria in the kidneys of the rats from the model group decreased, the arrangement was disordered, the morphology deformed and distorted, mild swelling occurred, the local matrix became lighter, the cristae expanded, and partial fractures disappeared. SZ-A can improve the above-mentioned mitochondrial lesions. ATP and mitochondrial DNA copy number in kidney tissues of the rats were detected, and the results showed (as shown in F and G in Fig. 12) that SZ-A could significantly improve ATP deficiency and mitochondrial copy number reduction in kidneys of the CKD rats, and alleviate mitochondrial damage.

### Experimental Example 5

Human renal tubular epithelial cells HK2 induced by TGF β1 (transforming growth factor β1) were treated with mulberry extracts from Preparative Examples 1 to 8. Further, the effect of mulberry extracts on the proliferation of HK2 cells and the effects of mulberry extracts on the synthesis of type IV collagen, fibronectin, and α-SAM gene transcription levels in HK2 cells were studied. The results showed that the mulberry extracts from the above preparative examples at the same dosage had varying degrees of effects.

### Industrial Application

The mulberry extract of the present invention, as ingredients derived from natural plants, has the unique advantages of low toxic side effect and mild and long-lasting effects. Therefore, mulberry extract with greatly improved medication safety has great advantages in the treatment of the chronic kidney disease.

## Claims

1. Any of the following uses (a1)-(a4) of a mulberry extract or main active ingredients thereof:
(a1) use of the mulberry extract in the preparation of a medicament for treating a chronic kidney disease;
(a2) use of the mulberry extract in the preparation of a medicament for preventing a chronic kidney disease;
(a3) use of the mulberry extract in the treatment of a chronic kidney disease; and
(a4) use of the mulberry extract in the prevention of a chronic kidney disease.

2. The use according to claim 1, **characterized in that** the chronic kidney disease comprises a diabetic kidney disease and/or a non-diabetic chronic kidney disease.

3. The use according to claim 1, **characterized in that** the prevention of the chronic kidney disease or treatment of the chronic kidney disease is reflected in at least one of:
1) improvement in renal function indexes in a subject with the chronic kidney disease;
2) improvement in oxidative/nitrative stress and inflammatory status in a subject with the chronic kidney disease;
3) reduction in levels of renal inflammatory factors in a subject with the chronic kidney disease;
4) improvement in renal pathological changes in a subject with the chronic kidney disease;
5) improvement in renal fibrosis in a subject with the chronic kidney disease;
6) reduction in a trend of blood creatinine in a subject with the chronic kidney disease;
7) alleviation in a trend of increasing ratio of kidney weight to body weight in a subject with the chronic kidney disease; and
8) improvement in renal inflammation in a subject with the chronic kidney disease.

4. Any of the following uses (b1)-(b12) of a mulberry extract or main active ingredients thereof:
(b1) preparation of a product for improving renal function indexes in a subject with a chronic kidney disease;
(b2) preparation of a product for improving oxidative/nitrative stress and inflammatory status in a subject with a chronic kidney disease;
(b3) preparation of a product for reducing levels of renal inflammatory factors in a subject with a chronic kidney disease;
(b4) preparation of a product for improving renal pathological changes in a subject with a chronic kidney disease;
(b5) preparation of a product for improving renal fibrosis in a subject with a chronic kidney disease;
(b6) preparation of a product for reducing a trend of blood creatinine in a subject with a chronic kidney disease
(b7) improvement in renal function indexes in a subject with a chronic kidney disease;
(b8) improvement in oxidative/nitrative stress and inflammatory status in a subject with a chronic kidney disease;
(b9) reduction in levels of renal inflammatory factors in a subject with a chronic kidney disease;
(b10) improvement in renal pathological changes in a subject with a chronic kidney disease;
(b11) improvement in renal fibrosis in a subject with a chronic kidney disease; and
(b12) reduction in a trend of blood creatinine in a subject with a chronic kidney disease.

5. The use according to claim 3 or 4, **characterized in that** the chronic kidney disease is a diabetic kidney disease;
the improvement in renal function indexes in the subject with the chronic kidney disease is reflected in at least one of: reduction in the level of blood urea nitrogen in the subject with the diabetic kidney disease; reduction in the contents of urinary albumin and β2-microglobulin in the subject with the diabetic kidney disease;
or, the improvement in oxidative/nitrative stress in the subject with the diabetic kidney disease is reflected in at least one of: a trend of increasing serum total antioxidant capacity and superoxide dismutase levels, significant reduction in serum inducible nitric oxide synthase and nitric oxide levels, reduction in renal mitochondrial lesion, and alleviation of mitochondrial damage in the subject with the diabetic kidney disease;
or, the improvement in inflammatory status in the subject with the diabetic kidney disease is specifically reflected in: the significant reduction in serum IL-1β and MCP-1 levels in the subject with the diabetic kidney disease;
or, the renal inflammatory factors include TNFα, IL-1β, IL-6, and C-reactive protein;
or, the renal pathological changes include at least one of: glomerular nodular sclerosis, varying degrees of renal interstitial fibrosis and inflammation, vascular hyalinization and sclerosis, and atrophy of renal tubular epithelial cells;
or, the renal fibrosis includes glomerular fibrosis and renal cystic fibrosis.

6. The use according to claim 3 or 4, **characterized in that** the chronic kidney disease is a non-diabetic chronic kidney disease;
the improvement in renal function indexes in the subject with the non-diabetic chronic kidney disease is specifically reflected in: reduction in the levels of serum creatinine and serum urea nitrogen in the subject with the non-diabetic chronic kidney disease;
or, the improvement in oxidative stress in the subject with the non-diabetic chronic kidney disease is specifically reflected in: improvement in the expression of SOD, CAT, and GSS, and reduction of the level of MDA in the subject with the chronic kidney disease;
or, the improvement in renal fibrosis in the subject with the non-diabetic chronic kidney disease is specifically reflected in: improvement in the expression of fibrosis related proteins in the subject with the chronic kidney disease; the fibrosis related proteins include at least one of: COL1A1, α-SMA, Fibronectine, and COL4A1;
or, the improvement in renal inflammation in the subject with the non-diabetic chronic kidney disease is specifically reflected in: inhibition of the infiltration of renal white blood cells and macrophages;
or, the renal pathological changes include at least one of the following aspects: atrophy or dilation of renal tubules, loss of brush borders, uneven thickening of renal basement membrane, and fusion or disappearance of podocytes.

7. The use according to any of claims 1-6, **characterized in that** the product is a medicament or a pharmaceutical formulation; and the mulberry extract is a Ramulus Mori extract, a Cortex Mori extract and/or a Folium Mori extract.

8. The use according to any of claims 1-7, **characterized in that**:
the method for preparing the mulberry extract comprises the steps of:
1) preparing a crude extract from a plant of *Moraceae*; and
2) separating the crude extract via a cation resin and/or optionally, an anion resin, to provide the mulberry extract.

9. The use according to claim 8, **characterized in that** the method further comprises the steps of:
3) subjecting the effluent from the resin in step 2) to alcohol precipitation and collecting a supernatant; and
4) concentrating and drying the supernatant;
alternatively, the method further comprises the step of: concentrating and drying the effluent from the resin in step 2).

10. The use according to any of claims 1-9, **characterized in that** the main active ingredients of the mulberry extract comprise at least one of: 1-deoxynojirimycin, N-methly-1-deoxynojirimycin, fagomine, 3-epi-fagomine, 1,4-dideoxy-1,4-imino-D-arabinitol, calystegin B2, calystegin C1, 2-O-(α -D-galactopyranosyl)-1-deoxynojirimycin, 6-O-(β-D-glucopyranosyl)-1-deoxynojirimycin, and 1,4-dideoxy-1,4-imino-(2-O-β -D-glucopyranosyl)-D-arabinitol;
alternatively, the mulberry extract acts on humans or mammals.
